# EUROPÄISCHE PATENTSCHRIFT

(11) **EP 0 589 355 B1**
(45) Veröffentlichungstag und Bekanntmachung des Hinweises auf die Patenterteilung: **29.12.1997**
(21) Anmeldenummer: 93114909.0
(22) Anmeldetag: 16.09.1993
(51) Int. Cl.: B65F 1/00, B65D 33/01, A61B 19/02, A61L 11/00, B01D 63/00

(54) **Müllsack für infektiösen Müll**
Refuse bag for infectious waste
Sac-poubelle pour déchets infectieux

(30) Priorität: 24.09.1992 DE 9212865 U
(43) Veröffentlichungstag der Anmeldung: 30.03.1994
(73) Patentinhaber: SENGEWALD KLINIKPRODUKTE GmbH, D-83101 Rohrdorf/Thansau (DE)
(72) Erfinder: Weimar, Albert, D-83026 Rosenheim (DE)
(74) Vertreter: Selting, Günther, Dipl.-Ing.

(56) Entgegenhaltungen:
- GB-A- 1 488 326
- US-A- 4 644 586
- US-A- 4 660 721
- US-A- 5 230 430

## Beschreibung

Die Erfindung betrifft einen Müllsack für infektiösen Müll und insbesondere einen Müllsack, der für Krankenhausmüll verwendbar ist.

Es ist bekannt, für Krankenhausmüll Kunststoffsäcke zu benutzen und die gefüllten Müllsäcke einem Sterilisationsprozeß zu unterwerfen, so daß die Keime im Inhalt des Müllsacks abgetötet werden und der zuvor infektiöse Müll wie Hausmüll entsorgt werden kann. Um bei der Dampfsterilisation den Zugang des Wasserdampfes zum Müll zu gewährleisten, muß der Sack offen sein. Der Sack wird in einen Stahlbehälter eingestellt, damit er in der Sterilisierkammer nicht umfällt. Der Stahlbehälter weist einen Filterdeckel auf, damit beim Vakuumziehen während des Sterilisationsprozesses der Müll den offenen Beutel nicht verläßt. Der Filterdeckel besteht aus gelochtem Stahlmaterial und einer porösen Schaumstoffmatte. Diese läßt nicht nur Luft und Wasserdampf durch, sondern auch den Geruch, so daß durch den erhitzten Infektionsmüll eine starke Geruchsbelästigung entsteht. Die aus Edelstahl bestehenden Stahlbehälter sind sehr teuer. Hinzukommen die Kosten für den Schaumstoffilter, der nach zwei bis drei Sterilisationen wegen nachlassender Durchlässigkeit ersetzt werden muß, und die Deckeldichtungen, die auch eine relativ kurze Lebensdauer haben. Die Stahlbehälter erfordern auch im leeren Zustand viel Platz. Sie müssen nach jedem Einsatz gereinigt werden und verursachen Logistikprobleme.

Die bisherigen Abfallsysteme für die Entsorgung infektiösen Mülls sind sehr aufwendig und teuer, umständlich im Handling und verursachen für das Personal kaum noch zumutbare Geruchsbelästigungen.

Bekannt sind ferner sterilisierbare Verpackungen für Medicalprodukte, die mit Gas sterilisiert werden. Diese Verpackungen bestehen aus Folienmaterial und sie haben an dem der Füllöffnung abgewandten Ende ein Wandteil aus luftdurchlässigem Filtermaterial. Nach dem Einbringen der Medicalprodukte werden die Beutel verschweißt und anschließend erfolgt eine Gassterilisation, wobei Beutel und Inhalt sterilisiert werden.

Aus US-A-4 644 586 ist ein Müllsack nach dem Oberbegriff des Patentanspruchs 1 bekannt, bei dem ein luftdurchlässiges keimdichtes Filtermaterial sich über die gesamte Länge beider Wände des Müllsacks erstreckt. Dieses Filtermaterial ist in der unteren Hälfte des Müllsacks außen mit einer für Flüssigkeit undurchlässigen Kunststoffolie bedeckt. Dadurch wird der Müllsack im unteren Bereich wasserundurchlässig, während er im oberen Bereich für ein Sterilisationsmittel durchlässig, jedoch für Bakterien undurchlässig ist. Bei diesem Müllsack erstreckt sich also das Filtermaterial über sämtliche Wandflächen, so daß bei der Herstellung des Müllsacks ein großer Bedarf an Filtermaterial besteht.

Der Erfindung liegt die Aufgabe zugrunde, einen Müllsack für infektiösen Müll zu schaffen, der mit geringen Kosten herstellbar ist und eine Sterilisation und einwandfreie Entsorgung des Mülls ohne stärkere Geruchsbelästigung ermöglicht.

Die Lösung dieser Aufgabe erfolgt erfindungsgemäß mit den im Anspruch 1 angegebenen Merkmalen.

Der erfindungsgemäße Müllsack besteht aus Kunststoffolie, wobei in der der Öffnung benachbarten Hälfte des Sackkörpers mindestens ein Wandteil aus einem luftdurchlässigen keimdichten flexiblen Filtermaterial angeordnet ist, das mit der Folie verschweißt ist. Die Folie und das Filtermaterial sind bis mindestens 134° C hitzebeständig, so daß sie einer Dampfsterilisation ausgesetzt werden können.

Der Müllsack erlaubt das Sammeln infektiösen Mülls. Seine untere Hälfte besteht ausschließlich aus Folienmaterial, so daß Flüssigkeiten, die in dem Müll enthalten sind, nicht auslaufen können. Die der Einfüllöffnung benachbarte Hälfte des Sackkörpers enthält das Wandteil aus Filtermaterial. Da dieses Wandteil flexibel ist, kann es zusammen mit dem Folienmaterial in der Nähe der Einfüllöffnung zusammengerafft werden, um die Einfüllöffnung mit einem üblichen Sackverschluß, z.B. mit einem Band, zu verschließen. Ein wesentlicher Vorteil des Müllsacks besteht darin, daß die Sterilisation bei geschlossenem Müllsack erfolgen kann. Durch das luftdurchlässige Filtermaterial kann zunächst eine Vakuumabsaugung erfolgen, ohne daß der Sackinhalt aus dem Sack herausgesaugt wird. Bei der Dampfsterilisation, die bei 134° C durchgeführt wird, dringt der Dampf durch das Filtermaterial hindurch und tötet die im Innern des Müllsacks befindlichen Keime ab. Der Sack ist während der Dampfsterilisierung geschlossen und durch das feinporige Filtermaterial werden die meisten Geruchsstoffe zurückgehalten, so daß außerhalb des geschlossenen Müllsacks keine wesentliche Geruchsbelästigung eintritt. Im Anschluß an die Sterilisierung kann der Sack aus der Sterilisierkammer entnommen werden, ohne daß noch weitere Handhabungen nötig wären. Der Müllsack ist in diesem Stadium bereits verschlossen und braucht lediglich entsorgt zu werden.

Gemäß der Erfindung ist die Folie eine Mehrschicht-Folie, die außen eine bei höheren Temperaturen beständige Trägerschicht und innen eine bei geringeren Temperaturen schmelzende Siegelungsschicht aufweist. Die außenliegende Schicht des Müllsacks, die der höheren Temperaturbelastung ausgesetzt ist, besteht aus hochschmelzbarem Kunststoff, während die innenliegende Siegelungsschicht aus niedrigschmelzenderem Kunststoff besteht. Das Filtermaterial ist mit der inneren Siegelungsschicht verbunden, d.h. es ist gewissermaßen in die Innenseite des Müllsacks eingeschweißt und nicht in der bei Verpackungen üblichen Form auf die Außenseite aufgeschweißt.

Das Filtermaterial besteht aus einem siegelbaren Kunststoffvlies von hoher Flexibilität und hoher Reißfestigkeit, so daß das der Sacköffnung benachbarte Ende zusammengerafft und mit einem Sackbinder verschlossen werden kann, wenn das Zuschweißen des Müllsacks aus Handlinggründung nicht akzeptabel ist.

Im folgenden wird unter Bezugnahme auf die Zeichnungen ein Ausführungsbeispiel der Erfindung näher erläutert.

Es zeigen:
- Fig. 1: eine Frontansicht des Müllsacks,
- Fig. 2: einen Schnitt entlang der Linie II-II von Fig. 1,
- Fig. 3: in vergrößertem Maßstab eine Darstellung der Einzelheit III in Fig. 2 und
- Fig. 4: eine Ansicht des verschlossenen Müllsacks.

Der Müllsack weist einen Sackkörper 10 von rechteckiger Form auf, der aus zwei aufeinandergelegten Folienteilen 11 und 12 besteht. Die Folienteile 11 und 12 sind entlang von drei Rändern durch Schweißnähte 13,14 und 15 miteinander verbunden. Die Schweißnaht 14 bildet die Bodennaht des Müllsacks und die Schweißnähte 13 und 15 bilden die Seitennähte. Das Folienteil 11 erstreckt sich über die gesamte Höhe des Müllsacks, während das Folienteil 12 sich nur etwa über die Hälfte bis zwei Drittel der Höhe des Müllsacks erstreckt. Nach oben ist das Folienteil 12 durch ein Wandteil 16 aus Filtermaterial verlängert. Dieses Wandteil 16 ist an die Innenseite des Folienteils 12 mit einer Schweißnaht 17 angeschweißt. Der obere Rand des Folienteils 11 bildet mit dem oberen Rand des Wandteils 16 die Einfüllöffnung 18 des Müllsacks. An den Seitenkanten ist das Wandteil 16 durch Schweißnähte 13a und 15a mit dem rückwärtigen Folienteil 11 verbunden.

Der Sackkörper 10 hat eine Höhe von etwa 90 cm und eine Breite von etwa 60 cm.

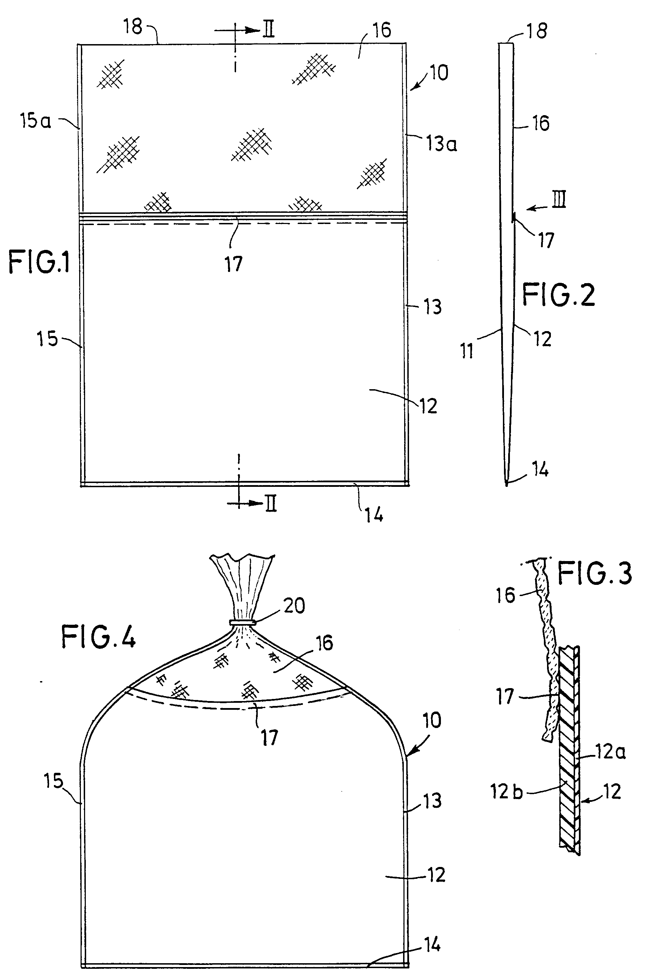

Wie Fig. 3 zeigt, ist die Folie 12 eine Mehrschichtfolie, die aus der dünneren Trägerschicht 12a und der dickeren Siegelungsschicht 12b besteht. Die Siegelungsschicht 12b ist auf der Innenseite der Trägerschicht 12a angeordnet, die beim Siegelungsvorgang einen Hitzeschild für die Siegelungsschicht bildet. In gleicher Weise ist die Folie 11b ausgebildet.

Die Trägerschicht 12a besteht aus einem hochschmelzbaren Kunststoff und die Siegelungsschicht 12b besteht aus einem anderen Kunststoff, der weniger weit oberhalb 134° C schmilzt.

Das Filtermaterial des Wandteils 16 besteht aus einem feinfaserigen Vlies, das punktgebondet ist. Ein solches Faservlies hat eine hohe Flexibilität und eine hohe Reißfestigkeit. Da das Wandteil 16 aus dem gleichen Grundmaterial besteht wie die Siegelungsschicht der Folienteile 11 und 12, kann es, z.B. in der Schweißnaht 17, sehr leicht mit den Folienteilen verschweißt werden.

Fig. 4 zeigt den gefüllten Sackkörper 10, dessen oberes Ende im Bereich des Wandteils 16 zusammengerafft und mit einem Sackbinder 20 verschlossen ist. Die untere Hälfte des Müllsacks besteht ausschließlich aus den miteinander verschweißten Folienteilen 11 und 12 und ist flüssigkeitsdicht. In der oberen Hälfte des Müllsacks befindet sich auf der einen Seite das luftdurchlässige keimdichte flexible Wandteil 16, das, mit Ausnahme der Füllöffnung 18, mit den angrenzenden Folienteilen 11 und 12 verschweißt ist und unterhalb des Sackbinders 20 eine Filterwand bildet.

## Patentansprüche

1. Müllsack für infektiösen Müll, bestehend aus einem an einem Ende offenen Sackkörper (10) aus Kunststoffolie, wobei ein in der der Öffnung (18) benachbarten Hälfte des Sackkörpers (10) befindliches Wandteil (16) aus einem luftdurchlässigen keimdichten flexiblen Filtermaterial besteht und ein von der Öffnung (18) entferntes Wandteil eine für Flüssigkeit undurchlässige Kunststoffolie aufweist, und die Folie und das Filtermaterial hitzebeständig sind,
**dadurch gekennzeichnet,**
daß die Folie und das Filtermaterial bis mindestens 134 °C hitzebeständig sind, und
daß das Filtermaterial entlang einer Schweißnaht (17) mit der Folie verschweißt ist und die Folie eine Mehrschicht-Folie ist, die an der Außenseite des Sackkörpers (10) eine Trägerschicht (12a) von hoher Schmelztemperatur und an der Innenseite eine Siegelungsschicht (12b) von niedrigerer Schmelztemperatur aufweist, und daß das Filtermaterial an die Innenseite der Folie (12) angeschweißt ist.

2. Müllsack nach Anspruch 1, dadurch gekennzeichnet, daß die Siegelungsschicht (12b) und das Filtermaterial aus dem gleichen Kunststoff bestehen.

3. Müllsack nach Anspruch 1 oder 2, dadurch gekennzeichnet, daß das Filtermaterial nur in einer der beiden Wände vorhanden ist.

4. Müllsack nach einem der Ansprüche 1 oder 2, dadurch gekennzeichnet, daß Wandteile aus Filtermaterial an beiden Wänden des Beutels vorhanden sind.

5. Müllsack nach einem der Ansprüche 1 bis 4, dadurch gekennzeichnet, daß das Filtermaterial aus einem punktgebondeten Faservlies besteht.

6. Müllsack nach einem der Ansprüche 1 bis 5, dadurch gekennzeichnet, daß der Sackkörper (10) zwei durch drei Schweißnähte (13,14,15) miteinander verbundene Folienteile (11,12) aufweist, von denen das eine kürzer ist als das andere, und daß das eine Folienteil (12) durch das Filtermaterial auf die Länge des anderen Folienteils (11) verlängert ist.

## Claims

1. A waste bag for infectious waste, comprising a bag body (10) made from a film of a synthetic material and being open on one end, wherein a wall portion (16) arranged in that half of said bag body (10) which is adjacent to said opening (18) consists of an air-permeable, germ-tight, flexible filtering material and a wall portion remote from said opening (18) comprises a film of a synthetic material impermeable to liquid, and said film and said filtering material are heat-resistant,
**characterized in**
that said film and said filtering material are heat-resistant up to at least 134°C, and that said filtering material is welded to said film along a welding seam (17), said film being a multi-layered film comprising, on the outer side of said bag body (10), a support layer (12a) having a high melting temperature and, on the inner side, a sealing layer (12b) having a lower melting temperature, and that said filtering material is welded to the inner side of said film (12).

2. The waste bag according to claim 1, characterized in that said sealing layer (12b) and said filtering material are made from the same synthetic material.

3. The waste bag according to claim 1 or 2, characterized in that said filtering material is provided only in one of the two walls.

4. The waste bag according to claim 1 or 2, characterized in that wall portions made from said filtering material are provided on both walls of said bag.

5. The waste bag according to claims 1 to 4, characterized in that said filtering material comprises a spot-bonded fibrous non-woven.

6. The waste bag according to claims 1 to 5, characterized in that said bag body (10) comprises two film portions (11, 12) connected to each other by three welding seams (13, 14,15), one of said film portions being shorter than the other and said one film portion (12) being lengthened by said filtering material up to the length of the other film portion (11).

## Revendications

1. Sac-poubelle pour déchets infectieux, se composant d'un corps de sac (10), ouvert à une extrémité, formé d'une feuille en matière plastique, dans lequel une partie de paroi (16), située dans la moitié du corps de sac (10) voisine de l'ouverture (18), est constituée d'une matière filtrante souple, étanche aux germes et perméable à l'air, et une partie de paroi, distante de l'ouverture (18), présente une feuille en matière plastique étanche aux liquides, la feuille et la matière filtrante résistant à la chaleur, caractérisé en ce que la feuille et la matière filtrante sont résistantes à la chaleur jusqu'à au moins 134°C, en ce que la matière filtrante est soudée à la feuille par une soudure continue (17) et en ce que la feuille est une feuille à plusieurs couches, qui présente, du côté extérieur du corps de sac (10), une couche-support (12a) à haute température de fusion et, du côté intérieur, une couche d'étanchéité (12b) à plus basse température de fusion, et en ce que la matière filtrante est fixée par soudage sur la face interne de la feuille (12).

2. Sac-poubelle selon la revendication 1, caractérisé en ce que la couche d'étanchéité (12b) et la matière filtrante sont faites de la même matière plastique.

3. Sac-poubelle selon l'une des revendications 1 ou 2, caractérisé en ce que la matière filtrante n'est présente que dans l'une des deux parois.

4. Sac-poubelle selon l'une des revendications 1 ou 2, caractérisé en ce que les parties de paroi en matière filtrante existent au niveau des deux parois du sac.

5. Sac-poubelle selon l'une des revendications 1 à 4, caractérisé en ce que la matière filtrante consiste en un non-tissé fixé par points.

6. Sac-poubelle selon l'une des revendications 1 à 5, caractérisé en ce que le corps de sac (10) présente deux parties en feuille (11, 12) reliées l'une à l'autre par trois soudures continues (13, 14, 15), une partie étant plus courte que l'autre, et en ce qu'une partie en feuille (12) est prolongée par la matière filtrante pour correspondre à la longueur de l'autre partie en feuille (11).
